# EUROPÄISCHE PATENTSCHRIFT

(11) **EP 1 572 038 B1**
(45) Veröffentlichungstag und Bekanntmachung des Hinweises auf die Patenterteilung: **05.03.2008**
(21) Anmeldenummer: 02782617.1
(22) Anmeldetag: 17.12.2002
(51) Int. Cl.: A61F 2/44

(54) **ZWISCHENWIRBELIMPLANTAT MIT KARDANISCH GELAGERTEN GELENKTEILEN**
INTERVERTEBRAL IMPLANT COMPRISING JOINT PARTS THAT ARE MOUNTED TO FORM A UNIVERSAL JOINT
IMPLANT INTERVERTEBRAL A ELEMENTS D'ARTICULATION LOGES A LA CARDAN

(43) Veröffentlichungstag der Anmeldung: 14.09.2005
(73) Patentinhaber: Synthes GmbH, 4436 Oberdorf (CH)
(72) Erfinder: AEBI, Max, Montreal, Quebec H3A 1A1 (CA); BURKARD, Dominique, CH-5014 Gretzenbach (CH); FRIGG, Robert, CH-2544 Bettlach (CH); LECHMANN, Beat, CH-2544 Bettlach (CH); MATHYS, Robert, Jr., CH-2544 Bettlach (CH); PAVLOV, Paul, NL-6523 NA Nijmegen (NL)
(74) Vertreter: Lusuardi, Werther
(86) Internationale Anmeldenummer: PCT/CH2002/000706
(87) Internationale Veröffentlichungsnummer: WO 2004/054477

(56) Entgegenhaltungen:
- EP-A- 0 282 161
- WO-A-99/59492
- DE-A- 19 750 382
- US-A1- 2002 052 656

## Beschreibung

Die Erfindung bezieht sich auf ein Zwischenwirbelimplantat gemäss dem Oberbegriff des Patentanspruchs 1 und auf ein Verfahren zum Ersetzen einer defekten, natürlichen Bandscheibe durch ein Zwischenwirbelimplantat gemäss dem Anspruch 19.

Nach Entfernung einer beschädigten, natürlichen Bandscheibe oder eines beschädigten Nukleus pulposus einer Bandscheibe werden Implantate oder Prothesen in den Zwischenwirbelraum zweier benachbarter Wirbelkörper eingebracht. Dabei entsteht das Ziel, wieder möglichst natürliche Zustände herbeizuführen, d.h. insbesondere die ursprüngliche Bandscheibenhöhe und damit den ursprünglichen Abstand zwischen den beiden benachbarten Wirbelkörpern wiederherzustellen. Ferner sollen Bewegungen der benachbarten Wirbelkörper relativ zueinander möglichst ohne Behinderung in ihrer natürlichen Art ausführbar sein. Hierzu ist die Erhaltung der Bewegungsmöglichkeiten bei einer Vorwärts/Rückwärtsneigung, d.h. Flexion und Extension der Wirbelkörper sowie bei einer lateralen Beugung der Wirbelkörper innerhalb der natürlichen Grenzen wesentlich. Die natürlichen Bänder und Muskeln entlang der Wirbelsäule werden im wesentlichen intakt gelassen, so dass diese die Bewegungen eines mechanischen Bandscheibenersatzes weiter stabilisieren.

Ein Zwischenwirbelimplantat dem Oberbegriff von Anspruch 1 ist aus der WO 99/59492 bekannt.

Eine gattungsgemässe Bandscheibenendoprothese ist aus der DE-A 35 29 761 BÜTTNER bekannt. Diese bekannte Bandscheibenendoprothese besteht im wesentlichen aus zwei symmetrischen Abschlussplatten mit gegeneinander gerichteten konkaven Gleitflächen und je einer aussenstehenden Oberfläche zur Anlage an die Grundplatte, respektive die Deckplatte der angrenzenden Wirbelkörper und einem zwischen den Abschlussplatten positionierten Distanzstück mit zu den konkaven Gleitflächen an den Abschlussplatten komplementär ausgestalteten konvexen Gleitflächen. Die Gleitflächen sind in einer Ausführungsform als Teilflächen einer Zylindermantelfläche ausgebildet, wobei die an den beiden Abschlussplatten angeordneten Gleitflächen komplementär zu je einer der angrenzenden Gleitflächen am Distanzstück ausgestaltet sind und je zwei komplementäre Gleitflächen die aufeinander verschiebbaren Artikulationsflächen eines um eine Drehachse rotierbaren Gelenkteiles bilden. Das Gelenk umfasst ein oberes und ein unteres Gelenkteil mit je einer Drehachse. Die beiden Drehachsen sind um 90° zueinander versetzt. Nachteilig an dieser bekannten Bandscheibenendoprothese ist, dass
a) den durch die natürliche Bandscheibe übertragbaren überlagerten Schwenkbewegungen insbesondere bei anterior-posterior und lateraler Flexion, welche bei der natürlichen Bandscheibe unabhängig voneinander sind, durch die Ausgestaltung einer Bandscheibenendoprothese mit nur einem Drehzentrum nicht Rechnung getragen wird;
b) durch Scherbewegungen, insbesondere bei Translation in anterior-posteriorer Richtung das Wirbelgelenk (Facettengelenk) belastet wird, wodurch für den Patienten Schmerzen verursacht werden können;
c) nachteilige Reibungskräfte bei zwei aufeinander gleitbaren, artikulierenden Flächen entstehen. Ferner sind an den Flächen Verschleiss, d.h. unter anderem auch Abrieb sowie Widerstand bei der Bewegung der Gelenkteile die Folge. Zudem besteht das Risiko des "Stick-Slip" Effektes;
d) ein mechanischer Bandscheibenersatz die weitere Degeneration der betroffenen Bewegungssegmente kaum aufhalten kann. Das Wiederherstellen der ursprünglichen Bewegungsverhältnisse reduziert den Schmerz wesentlich und der Patient gewinnt an Lebensqualität. Bei neuem Auftreten von Schmerz muss jedoch eine Revision der Versorgung in Angriff genommen werden. Dabei wird üblicherweise eine Bandscheibenprothese nach herkömmlicher Bauart komplett entfernt und das Bewegungssegment versteift. Diese Operation belastet den Patienten ausserordentlich; und
e) der Form der Kontaktflächen zu den benachbarten Wirbelkörpern in der Regel nicht Rechnung getragen wird. Bandscheibenersatzimplantate herkömmlicher Bauart haben plane (flache) Kontaktflächen, welche oft noch mit kielartigen Erhebungen ergänzt sind.

Hier will die Erfindung Abhilfe schaffen. Der Erfindung liegt die Aufgabe zugrunde, ein Zwischenwirbelimplantat zu schaffen, welches Gelenke mit minimalen Reibungsflächen aufweist.

Die Erfindung löst die gestellte Aufgabe mit einem Zwischenwirbelimplantat, welches die Merkmale des Anspruchs 1 aufweist.

Die durch die Erfindung erreichten Vorteile sind im wesentlichen darin zu sehen, dass dank des erfindungsgemässen Zwischenwirbelimplantates
- die Schwenkbewegungen in anterior-posteriorer Richtung und nach lateral unabhängig voneinander sind;
- keine Translationsbewegungen der angrenzenden Wirbelkörper zugelassen wird, wodurch die Facettengelenke geschont werden; und
- die Reibungsflächen der bewegten Elemente auf kleine zylindrische oder polygonförmige Rotationskörper beschränkt ist und daher auf einem Minimum gehalten wird.

In einer bevorzugten Ausführungsform des erfindungsgemässen Zwischenwirbelimplantates umfassen die beiden Gelenke drei Gelenkteile, wobei das mittlere Gelenkteil als Rahmen ausgestaltet ist und dieser Rahmen einerseits mittels zwei koaxial zur ersten Drehachse angeordneten Achsen um die erste Drehachse rotierbar mit dem unteren Gelenkteil verbunden ist, und andererseits mittels einer weiteren, koaxial zur zweiten Drehachse angeordneten Achse um die zweite Drehachse rotierbar mit dem oberen Gelenkteil verbunden ist. Dabei können die Drehachsen windschief oder in einer Ebene und sich schneidend angeordnet sein.

In einer weiteren Ausführungsform des erfindungsgemässen Zwischenwirbelimplantates ist das mittlere Gelenkteil kreuzförmig ausgestaltet.

In wiederum einer weiteren Ausführungsform des erfindungsgemässen Zwischenwirbelimplantates ist das mittlere Gelenkteil winkelförmig ausgestaltet. Damit ist pro Gelenk nur eine zur jeweiligen Drehachse koaxiale Achse notwendig, wodurch der Vorteil erreichbar ist, dass die beiden Gelenke durch weniger Bauteile realisierbar sind.

In einer anderen Ausführungsform des erfindungsgemässen Zwischenwirbelimplantates sind von den ventralen Seitenflächen her Mittel an den beiden Teilen anbringbar, wodurch die beiden Teile ventral auf einer festen Distanz relativ zueinander gehalten werden können. Dadurch ist der Vorteil erreichbar, dass die beiden Teile zur Einführung in den Zwischenwirbelraum in eine Position mit fest gehaltener Höhe bringbar sind und nach der Einführung in den Zwischenwirbelraum um das Gelenk bewegbar und an die Grund- respektive Deckplatte der angrenzenden Wirbelkörper zur Anlage bringbar sind.

Bei dem erfindungsgemässen Zwischenwirbelimplantat ermöglichen Mittel eine temporäre Blockierung der Beweglichkeit der beiden Teile um das Gelenk. Dadurch ist der Vorteil erreichbar, dass mittels eines minimal invasiven Eingriffes das im Zwischenwirbelraum integrierte Gelenk blockierbar ist. Dies ist besonders vorteilhaft in Fällen bei denen post-operativ Schmerzen auftreten, d.h. wo die Degeneration des betroffenen Wirbelsäulensegmentes weitergeht und der Chirurg eine Fusion der betroffenen Wirbel in Betracht zieht. Vorzugsweise sind die Mittel an den beiden ventralen Seitenflächen der beiden Teile anbringbar. Durch dieses spätere, sekundäre Blockieren der Bewegbarkeit der beiden Teile um das Gelenk wird das Zwischenwirbelimplantat versteift und in ein Arthrodesenimplantat (Fusions-Käfig) übergeführt.

In einer weiteren Ausführungsform des erfindungsgemässen Zwischenwirbelimplantates umfassen die Mittel zur Blockierung des Gelenkes, welche zwei Einsatzstücke umfassen. Die beiden Einsatzstücke sind mittels Schrauben parallel zur zweiten Drehachse an den unteren Gelenkteilen fixierbar. Bei eingesetzten Einsatzstücken werden das obere Teil und das untere Teil so aneinander abgestützt, dass eine Drehbewegung eines der Teile relativ zum anderen um die beiden Drehachsen ausgeschlossen ist.

Bei dem erfindungsgemässen Zwischenwirbelimplantat umfassen die Mittel einen Einsatz, welcher in je eine Vertiefung an den einander gegenüberliegenden Oberflächen des oberen und unteren Teiles einsetzbar ist. Vorzugsweise sind die Vertiefungen als Schwalbenschwanzführungen ausgestaltet, welche an den ventralen Seitenflächen offen sind, so dass die zu den Schwalbenschwanzführungen komplementär ausgestalteten Enden des Einsatzes von ventral in die Schwalbenschwanzführungen eingeschoben werden können. Dadurch ist der Vorteil erzielbar, dass durch das Einführen des Einsatzes die Bewegbarkeit der beiden Teile um das Gelenk blockierbar ist. Die Starrheit der Blockierung lässt sich erhöhen, wenn die Schwalbenschwanzführungen so ausgestaltet sind, dass sie sich gegen die Zentralachse des Zwischenwirbelimplantates verjüngen, so dass der Einsatz zusätzlich in den Schwalbenschwanzführungen verkeilbar ist.

In einer weiteren Ausführungsform des erfindungsgemässen Zwischenwirbelimplantates umfassen die Mittel zwei parallel Einsätze, welche parallel zu den lateralen Seitenflächen zwischen die beiden Teile schiebbar sind und an den einander gegenüberliegenden Oberflächen der beiden Teile zu Anlage kommen. Beide Einsätze sind am unteren Teil mittels je einer Schraube fixierbar.

In wiederum einer anderen Ausführungsform des erfindungsgemässen Zwischenwirbelimplantates sind die beiden Teile mit Bohrungen zur Aufnahme von Knochenfixationsmittel, insbesondere von Knochenschrauben versehen, wobei die Bohrungen Längsachsen aufweisen, welche schräg zur Zentralachse stehen. Vorzugsweise durchdringen je zwei Bohrungen eines der beiden Teile von der ventralen Seitenfläche zur Appositionsfläche. Dabei können die Längsachsen, falls nur eine axiale Fixierung des Zwischenwirbelimplantates vorgesehen ist, nur von lateral betrachtet schräg zur Zentralachse stehen, oder falls eine winkelstabile Fixierung des Zwischenwirbelimplantates vorgesehen ist, auch von ventral betrachtet von den inneren Oberflächen der beiden Teile gegen die Appositionsflächen divergieren.

In einer weiteren Ausführungsform des erfindungsgemässen Zwischenwirbelimplantates sind die Bohrungen zur Aufnahme der Knochenfixationsmittel mit Innengewinden versehen, wodurch sich eine zusätzliche, rigide Fixierung der Knochenfixationsmittel in den beiden Teilen erreichen lässt. Vorzugsweise sind die Bohrungen konisch ausgestaltet, so dass durch die konischen Gewindeverbindungen zwischen den Innengewinden und den Aussengewinden an den Köpfen der Knochenfixationsmittel eine verstärkte Fixierung der Knochenfixationsmittel an jedem der beiden Teile erreichbar ist.

Der Ersatz einer defekten, natürlichen Bandscheibe durch ein Zwischenwirbelimplantat umfasst die Schritte:
A) blockieren des oder der Gelenke eines Zwischenwirbelimplantates mittels dafür vorgesehener Mittel in einer bestimmten Position des oder der Gelenke;
B) einführen des Zwischenwirbelimplantates in den zu behandelnden Zwischenwirbelraum;
C) lösen und entfernen der zur Blockierung des oder der Gelenke in das Zwischenwirbelimplantat eingesetzten Mittel. Durch die Blockierung des Gelenkes ist der Vorteil erreichbar, dass die beweglichen Teile mit den aussenstehenden Appositionsflächen einfacher in den zu behandelnden Zwischenwirbelraum einführbar sind.

In einer weiteren Anwendung des Verfahren umfasst dieses das nachträgliche Blockieren des oder der Gelenke am implantierten Zwischenwirbelimplantat mittels der zur Blockierung des oder der Gelenke vorgesehenen Mittel. Dadurch ist der Vorteil erreichbar, dass bei einem Auftreten von post-operativen Schmerzen für den Patienten oder bei einer weiteren Degeneration des betroffenen Bewegungssegmentes das oder die Gelenke am Zwischenwirbelimplantat postoperativ durch Einsetzen der dazu vorgesehenen Mittel blockierbar sind. Diese nachträgliche Blockierung ist mit einem minimal-invasiven, vorzugsweise einem lapraskopischen Eingriff möglich. Das Zwischenwirbelimplantat übernimmt dann die Aufgabe eines Käfigs, so dass das betroffene Bewegungssegment der Wirbelsäule versteift werden kann.

Die Erfindung und Weiterbildungen der Erfindung werden im folgenden anhand der teilweise schematischen Darstellungen eines Ausführungsbeispieles noch näher erläutert.

Es zeigen:
Fig. 1 eine Explosionsdarstellung einer Ausführungsform des erfindungsgemässen Zwischenwirbelimplantates;
Fig. 2 eine perspektivische Ansicht der in Fig. 1 dargestellten Ausführungsform des erfindungsgemässen Zwischenwirbelimplantates in zusammengesetztem Zustand;
Fig. 3 eine Ansicht von lateral auf eine weitere Ausführungsform des erfindungsgemässen Zwischenwirbelimplantates; und
Fig. 4 eine perspektivische Ansicht von ventral auf die Ausführungsform nach Fig. 3.

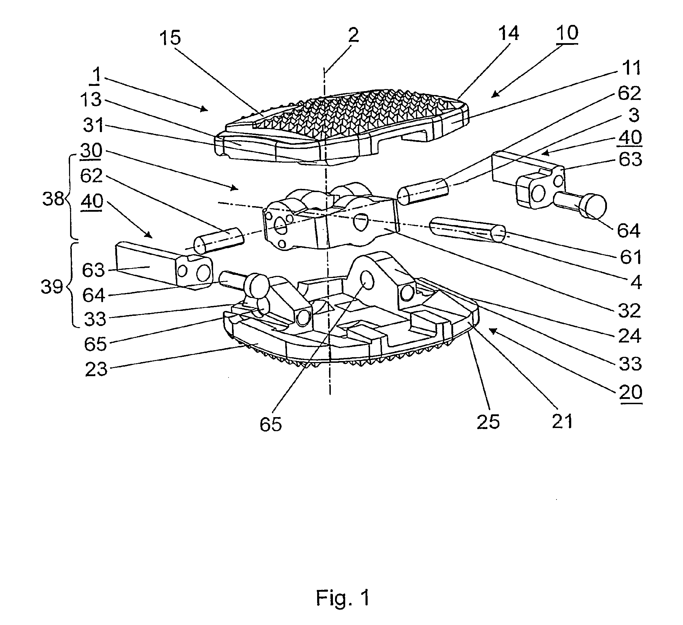

In Fig. 1 ist eine Ausführungsform des erfindungsgemässen Zwischenwirbelimplantates 1 dargestellt, welche ein oberes Teil 10 mit einer oberen, quer zur Zentralachse 2 angeordneten Appositionsfläche 15 zur Anlage an die Grundplatte eines angrenzenden Wirbelkörpers, ein unteres Teil 20 mit einer unteren, quer zur Zentralachse 2 angeordneten Appositionsfläche 25 zur Anlage an die Deckplatte des angrenzenden Wirbelkörpers und ein Gelenk 30 umfasst. Das obere Teil 10 und das untere Teil 20 sind über das Gelenk 30 relativ zueinander bewegbar verbunden, wobei die Bewegbarkeit des oberen Teils 10 relativ zum unteren Teil 20 um eine erste, quer zur Zentralachse 2 angeordnete Drehachse 3 innerhalb eines Winkelbereiches von +10° bis -6° eingeschränkt ist und um eine zweite, quer zur Zentralachse 2 und senkrecht zur ersten Drehachse 3 angeordneten Drehachse 4 innerhalb eines Winkelbereiches von ± 7° eingeschränkt ist.

Das Gelenk 30 ist als Kardangelenk ausgestaltet und umfasst ein als Rahmen ausgestaltetes, mittleres Gelenkteil 32 mit zwei koaxial zur ersten Drehachse 3 angeordneten Achsen 62, welche in zwei dazu komplementären Bohrungen 65 an den unteren Gelenkteilen 33 um die erste Drehachse 3 rotierbar gelagert sind. Eine weitere, koaxial zur zweiten Drehachse 4 angeordnete Achse 61 ist am mittleren Gelenkteil 32 angebracht und in einer dazu komplementären Bohrung (nicht gezeichnet) am oberen Gelenkteil 31 um die zweite Drehachse 4 rotierbar gelagert. Die Achsen 61;62 können im orthogonal zur Drehachse 3;4 betrachteten Querschnitt eine kreisförmige oder polygon artige Querschnittsfläche aufweisen. Die Blockierung des Gelenkes 30 erfolgt in der hier dargestellten Ausführungsform durch Mittel 40, welche zwei Einsatzstücke 63 umfassen, welche parallel zur zweiten Drehachse 4 an den unteren Gelenkteilen 33 mittels Schrauben 64 fixierbar sind. Bei eingesetzten Einsatzstücken 63 werden das obere Teil 10 und das untere Teil 20 so aneinander abgestützt, dass weder eine Drehbewegung eines der Teile 10;20 relativ zum anderen um die erste Drehachse 3, noch eine Drehbewegung eines der Teile 10;20 relativ zum anderen um die zweite Drehachse 4 möglich ist.

Die beiden Teile 10;20 sowie das mittlere Gelenkteil 32 werden durch die im mittleren Gelenkteil 32 fixierten Achsen 61;62, welche in den Bohrungen 65 im unteren Gelenkteil 33 und einer Bohrung (nicht gezeichnet) im oberen Gelenkteil 31 um die Drehachsen 3;4 rotierbar gelagert sind, zusammengehalten.

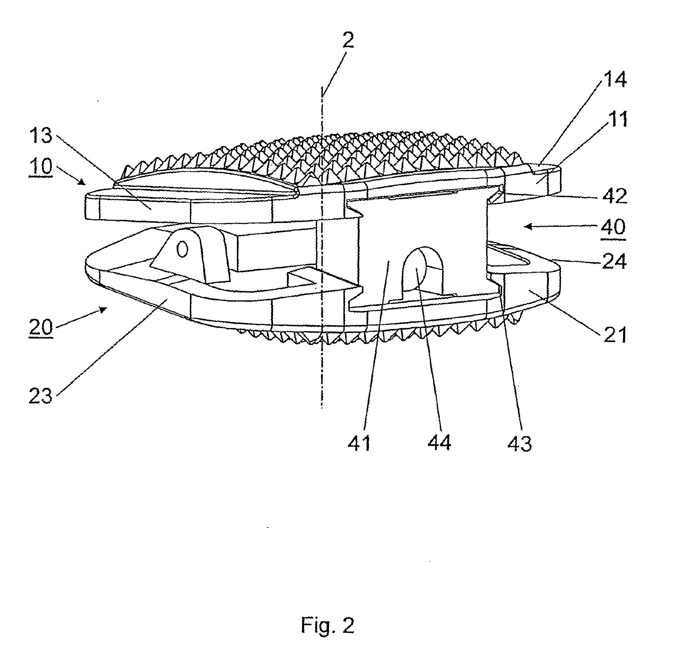

Die in Fig. 2 dargestellte Ausführungsform des erfindungsgemässen Zwischenwirbelimplantates unterscheidet sich von der in Fig. 1 dargestellten Ausführungsform nur darin, dass die Mittel 40 anders ausgestaltet sind. Die Mittel 40 umfassen in der hier dargestellten Ausführungsform einen von den ventralen Seitenflächen 11;21 der beiden Teile 10;20 her quer zur Zentralachse 2 und parallel zu den lateralen Seitenflächen 13;14;23;24 der beiden Teile 10;20 einschiebbaren Einsatz 41. Das Einschieben des Einsatzes 41 erfolgt in zwei Vertiefungen 42;43, welche als Schwalbenschwanzführungen ausgestaltet sind. Der Einsatz 41 wird von den ventralen Seitenflächen 11;21 der beiden Teile 10;20 in die als Schwalbenschwanzführungen ausgestalteten Vertiefungen 42;43 eingeführt und am unteren Teil 20 mittels einer Schraube 44 befestigt. Zudem ist der Einsatz 41 endständig komplementär zu den Vertiefungen 42;43 ausgestaltet, so dass die beiden Teile 10;20 bei eingeschobenem Einsatz 41 parallel zur Zentralachse 2 relativ zueinander fixiert sind.

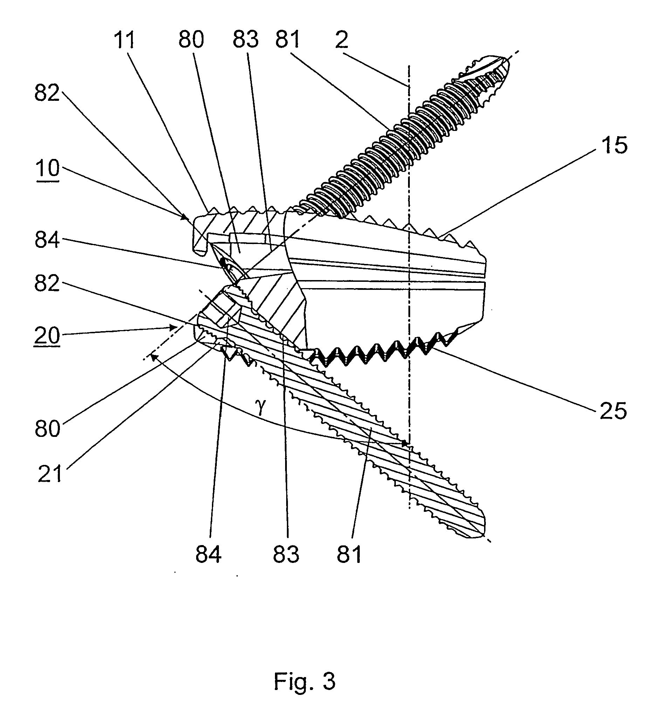

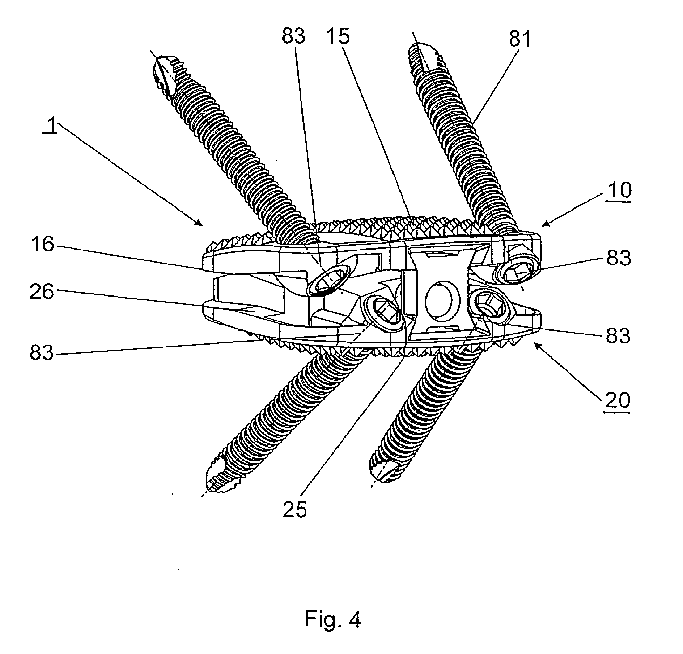

In Fig. 3 ist eine Ausführungsform des erfindungsgemässen Zwischenwirbelimplantates 1 dargestellt, welche sich von der in den Fig. 1 und 2 dargestellten Ausführungsform nur darin unterscheidet, dass die beiden Teilen 10;20 Bohrungen 80 zur Aufnahme von Knochenfixationsmitteln 81 umfassen, wobei die Knochenfixationsmittel 80 hier als Knochenschrauben ausgestaltet sind. Die Bohrungen 80 weisen Längsachsen 83 auf, welche einen Winkel γ mit der Zentralachse 2 einschliessen. Ferner durchdringen je zwei Bohrungen 80 (Fig. 4) eines der beiden Teile 10;20 von der ventralen Seitenfläche 11;21 zur Appositionsfläche 15;25. Die Längsachsen 83 der Bohrungen 80 stehen sowohl von lateral betrachtet (Fig. 3) als auch von ventral betrachtet (Fig. 4) schräg zur Zentralachse 2. Ferner sind die Bohrungen 80 konisch, sich gegen die Appositionsflächen 15;25 verjüngend ausgestaltet und mit Innengewinden 82 versehen, welche zur schraubbaren Aufnahme der mit komplementären Aussengewinden versehenen Schraubenköpfe 84 der als Knochenschrauben ausgestalteten Knochenfixationsmittel 81 dienen.

## Patentansprüche

1. Zwischenwirbelimplantat (1), insbesondere künstliche Bandscheibe, mit einer Zentralachse (2), einem oberen Teil (10), das für die Anlage an die Grundplatte eines darüber liegenden Wirbelkörpers geeignet ist und einem unteren Teil (20), das für die Anlage an die Deckplatte eines darunter liegenden Wirbelkörpers geeignet ist, wobei
A) das obere Teil (10) eine ventrale Seitenfläche (11), eine dorsale Seitenfläche (12), zwei laterale Seitenflächen (13,14), eine obere Appositionsfläche (15) und eine untere Oberfläche (16) aufweist;
B) das untere Teil (20) eine ventrale Seitenfläche (21), eine dorsale Seitenfläche (22), zwei laterale Seitenflächen (23,24), eine untere Appositionsfläche (25) und eine obere Oberfläche (26) aufweist;
C) die beiden Teile (10,20) durch zwei zwischen den beiden Teilen (10;20) angeordnete Gelenke (38;39) relativ zueinander bewegbar sind, wobei
D) jedes der Gelenke (38;39) eine Drehachse (3;4) aufweist und die beiden Drehachsen (3;4) quer zueinander angeordnet sind;
E) die beiden Gelenke (38;39) durch ein mit dem oberen Teil (10) verbundenes, oberes Gelenkteil (31), ein mittleres Gelenkteil (32) und ein mit dem unteren Teil (20) verbundenes, unteres Gelenkteil (33) realisiert sind, wobei
F) das mittlere Gelenkteil (32) mittels mindestens einer zur Drehachse (3) koaxialen Achse (62) um die Drehachse (3) rotierbar mit dem unteren Gelenkteil (33) und mittels mindestens einer zur Drehachse (4) koaxialen Achse (61) um die Drehachse (4) rotierbar mit dem oberen Gelenkteil (31) verbunden ist,
**dadurch gekennzeichnet, dass**
G) dass Mittel (40) vorgesehen sind, welche geeignet sind eine temporäre Blockierung der Beweglichkeit der beiden Teile (10,20) um das Gelenk (30) herbeizuführen, wobei
H) die Mittel (40) einen Einsatz (41) mit einem unteren Ende und einem oberen Ende und an den beiden Teilen (10;20) je eine Vertiefung (42;43) in den Oberflächen (16;26) umfassen, welche an den ventralen Seitenflächen (11;21) offen sind, und
l) der Einsatz (41) mit seinen Enden in je eine Vertiefung (42;43) einfügbar ist.

2. Zwischenwirbelimplantat (1) nach Anspruch 1, **dadurch gekennzeichnet, dass** das mittlere Gelenkteil (32) als Rahmen ausgestaltet ist.

3. Zwischenwirbelimplantat (1) nach Anspruch 1, **dadurch gekennzeichnet, dass** das mittlere Gelenkteil (32) kreuzförmig ausgestaltet ist.

4. Zwischenwirbelimplantat (1) nach Anspruch 1, **dadurch gekennzeichnet, dass** das mittlere Gelenkteil (32) winkelförmig ausgestaltet ist.

5. Zwischenwirbelimplantat (1) nach einem der Ansprüche 1 bis 4, **dadurch gekennzeichnet, dass** Mittel (40) vorgesehen sind, welche die beiden Teile (10;20), bei ihren ventralen Seitenflächen (11;21) gemessen, auf einer festen Distanz voneinander halten.

6. Zwischenwirbelimplantat (1) nach einem der Ansprüche 1 bis 5, **dadurch gekennzeichnet, dass** die Mittel (40) an den beiden ventralen Seitenflächen (11,21) der beiden Teilen (10;20) anbringbar sind.

7. Zwischenwirbelimplantat (1) nach einem der Ansprüche 1 bis 6, **dadurch gekennzeichnet, dass** die Vertiefungen (42;43) an den beiden Teilen (10;20) Schwalbenschwanzführungen sind und die Enden am Einsatz (41) komplementär zu diesen Schwalbenschwanzführungen ausgestaltet sind.

8. Zwischenwirbelimplantat (1) nach Anspruch 7, **dadurch gekennzeichnet, dass** sich die Schwalbenschwanzführungen von den ventralen Seitenflächen (11;21) her gegen die dorsalen Seitenflächen (12;22) verjüngen.

9. Zwischenwirbelimplantat (1) nach einem der Ansprüche 1 bis 8, **dadurch gekennzeichnet, dass** die Mittel (40) zwei zu den lateralen Seitenflächen (13;14;23;24) parallele Einsatzstücke (63) umfassen, welche an den einander gegenüberliegenden Oberflächen (16;26) zu Anlage bringbar sind.

10. Zwischenwirbelimplantat (1) nach einem der Ansprüche 1 bis 9, **dadurch gekennzeichnet, dass** der Einsatz (41) mittels einer Schraube (44) an einem der beiden Teile (10;20) lösbar fixierbar ist.

11. Zwischenwirbelimplantat (1) nach einem der Ansprüche 1 bis 10, **dadurch gekennzeichnet, dass** das obere und das untere Teil (10;20) je mindestens zwei von den ventralen Seitenflächen (11;21) zu den Appositionsflächen (15;25) durchgehende Bohrungen (80) mit Längsachsen (83) zur Aufnahme von Knochenfixationsmitteln (81) umfassen.

12. Zwischenwirbelimplantat (1) nach Anspruch 11, **dadurch gekennzeichnet, dass** die Längsachsen (83) der Bohrungen (80) mit der Zentralachse (2) einen Winkel γ einschliessen.

13. Zwischenwirbelimplantat (1) nach Anspruch 12, **dadurch gekennzeichnet, dass** der Winkel γ in einem Bereich von 20° und 65° liegt.

14. Zwischenwirbelimplantat (1) nach einem der Ansprüche 11 bis 13, **dadurch gekennzeichnet, dass** die Längsachsen (83) der Bohrungen (80) von den ventralen Seitenflächen (11;21) aus betrachtet von den inneren Oberflächen (16;26) gegen die Appositionsflächen (15;25) divergieren.

15. Zwischenwirbelimplantat (1) nach einem der Ansprüche 11 bis 14, **dadurch gekennzeichnet, dass** sich die Bohrungen (80) gegen die Appositionsflächen (15;25) konisch verjüngen.

16. Zwischenwirbelimplantat (1) nach einem der Ansprüche 11 bis 15, **dadurch gekennzeichnet, dass** die Bohrungen (80) ein Innengewinde (82) aufweisen.

## Claims

1. Intervertebral implant (1), specifically an artificial intervertebral disk, with a central axis (2), an upper section (10), suitable for laying onto the base plate of a vertebral body lying above and a lower section (20) suitable for laying onto the cover plate of a vertebral body lying below, wherein
A) the upper section (10) is provided with a ventral side area (11), a dorsal side area (12), two lateral side areas (13,14), a top apposition surface (15) and a bottom surface (16);
B) the lower section (20) is provided with a ventral side area (21), a dorsal side area (22), two lateral side areas (23,24), a bottom apposition surface (25) and a top surface (26);
C) the two sections (10,20) moveable in relation to each other by means of two joints (38;39) arranged between the two sections (10;20), wherein
D) each of the joints (38;39) is provided with a swivel axle (3;4) and the two swivel axles (3;4) are arranged transversely to each other;
E) the two joints (38;39) are realised by means of an upper joint element (31) connected with the upper section (10), a central joint element (32) and a lower joint element (33) connected with the lower section (20), whereby
F) the central joint section (32) is connected with the lower joint section (33) by means of at least one axle (62) coaxial to the swivel axle (3) and rotatable around the swivel axle (3) and with the upper joint section (31) by means of at least one axle (61) coaxial to the swivel axle (4) and rotatable around the swivel axle (4).
**characterised in that**
G) a means (40) is provided that is suitable for causing temporary blocking of the mobility of the two sections (10,20) around the joint (30), whereby
H) the means (40) comprises an insert (41) with a lower end and an upper end and a depression (42;43) in the surfaces (16;26) at each of the two sections (10;20), which are open on the ventral side areas (11;21), and
I) the insert (41) with its ends can be inserted into each of the depressions (42;43).

2. Intervertebral implant (1) according to Claim 1, **characterised in that** the central joint element (32) is provided in the form of a frame.

3. Intervertebral implant (1) according to Claim 1, **characterised in that** the central joint section (32) is designed in the form of a cross.

4. Intervertebral implant (1) according to Claim 1, **characterised in that** the central joint section (32) is designed in the form of an angle.

5. Intervertebral implant (1) according to one of the Claims 1 to 4, **characterised in that** a means (40) is provided that keeps the two sections (10;20), measured at their ventral side areas (11;21), at a fixed distance from each other.

6. Intervertebral implant (1) according to one of the Claims 1 to 5, **characterised in that** the means (40) can be attached to the two ventral side areas (11,21) of the two sections (10;20).

7. Intervertebral implant (1) according to one of the Claims 1 to 6, **characterised in that** the depressions (42;43) are dovetail guides and the ends on the insert (41) are arranged complementary to these dovetail guides.

8. Intervertebral implant (1) according to Claim 7, **characterised in that** the dovetail guides are tapered from the ventral side areas (11;21) towards the dorsal side areas (12;22).

9. Intervertebral implant (1) according to one of the Claims 1 to 8, **characterised in that** the means (40) comprises two insert pieces (63) parallel to the lateral side surfaces (13;14;23;24), which can be attached to the surfaces (16;26) facing each other.

10. Intervertebral implant (1) according to one of the Claims 1 to 9, **characterised in that** the insert (41) can be attached to one of the two sections (10;20) by means of a screw (44) in a way that can be released.

11. Intervertebral implant (1) according to one of the claims 1 to 10, **characterised in that** the upper and the lower sections (10;20) each comprises at least two drill holes (80) running through from the ventral side areas (11;21) to the apposition surfaces (15;25) with longitudinal axes (83) for receiving bone fixation devices (81).

12. Intervertebral implant (1) according to Claim 11, **characterised in that** the longitudinal axes (83) of the drill holes (80) make an angle *γ* with the central axis (2).

13. Intervertebral implant (1) according to Claim 12, **characterised in that** the angle γ lies in a range of between 20° and 65°.

14. Intervertebral implant (1) according to one of the claims 11 to 13, **characterised in that** the longitudinal axes (83) of the drill holes (80) as seen from the ventral side areas (11;21) diverge from the inner surfaces (16;26) against the apposition surfaces (15;25).

15. Intervertebral implant (1) according to one of the claims 11 to 14, **characterised in that** the drill holes (80) are conically tapered towards the apposition surfaces (15;25).

16. Intervertebral implant (1) according to one of the claims 11 to 15, **characterised in that** the drill holes (80) are provided with an internal thread (82).

## Revendications

1. Implant intervertébral (1), en particulier disque intervertébral artificiel, présentant un axe central (2), une partie supérieure (10), qui est appropriée à l'appui contre le plateau inférieur d'un corps vertébral sus-jacent, et une partie inférieure (20), qui est appropriée à l'appui contre le plateau supérieur d'un corps vertébral sous-jacent, dans lequel
A) la partie supérieure (10) présente une face latérale ventrale (11), une face latérale dorsale (12), deux faces latérales sur le côté (13 ; 14), une surface d'apposition supérieure (15) et une surface inférieure (16) ;
B) la partie inférieure (20) présente une face latérale ventrale (21), une face latérale dorsale (22), deux faces latérales sur le côté (23 ; 24), une surface d'apposition inférieure (25) et une surface supérieure (26) ;
C) les deux parties (10 ; 20) sont mobiles l'une par rapport à l'autre au moyen de deux articulations (38 ; 39) disposées entre les deux parties (10 ; 20),
D) chacune des articulations (38 ; 39) présentant un axe de rotation (3 ; 4) et les deux axes de rotation (3 ; 4) étant disposés transversalement l'un à l'autre ;
E) les deux articulations (38 ; 39) étant formées par une partie d'articulation supérieure (31) reliée à la partie supérieure (10), une partie d'articulation intermédiaire (32) et une partie d'articulation inférieure (33) reliée à la partie inférieure (20),
F) la partie d'articulation intermédiaire (32) étant reliée à la partie d'articulation inférieure (33) de manière à pouvoir tourner autour de l'axe de rotation (3) au moyen d'au moins un pivot (62) coaxial à l'axe de rotation (3), et à la partie d'articulation supérieure (31) de manière à pouvoir tourner autour de l'axe de rotation (4) au moyen d'au moins un pivot (61) coaxial à l'axe de rotation (4),
**caractérisé en ce que**
G) des moyens (40) sont prévus qui sont appropriés pour provoquer un blocage temporaire de la mobilité des deux parties (10 ; 20) autour de l'articulation (30),
H) les moyens (40) comprenant une pièce rapportée (41) présentant une extrémité inférieure et une extrémité supérieure et, au niveau des deux parties (10 ; 20), un évidement (42 ; 43) dans les surfaces (16 ; 26) qui sont ouvertes au niveau des faces latérales ventrales (11 ; 21), et
l) la pièce rapportée (41) peut être insérée avec ses extrémités dans un évidement (42 ; 43).

2. Implant intervertébral (1) selon la revendication 1, **caractérisé en ce que** la partie d'articulation intermédiaire (32) est conçue sous forme de cadre.

3. Implant intervertébral (1) selon la revendication 1, **caractérisé en ce que** la partie d'articulation intermédiaire (32) est conçue en forme de croix.

4. Implant intervertébral (1) selon la revendication 1, **caractérisé en ce que** la partie d'articulation intermédiaire (32) est conçue sous forme angulaire.

5. Implant intervertébral (1) selon l'une des revendications 1 à 4, **caractérisé en ce que** des moyens (40) sont prévus qui maintiennent les deux parties (10 ; 20) à distance fixe l'une de l'autre, mesuré au niveau de leurs faces latérales ventrales (11 ; 21).

6. Implant intervertébral (1) selon l'une des revendications 1 à 5, **caractérisé en ce que** les moyens (40) peuvent être placés sur les deux faces latérales ventrales (11 ; 21) des deux parties (10 ; 20).

7. Implant intervertébral (1) selon l'une des revendications 1 à 6, **caractérisé en ce que** les évidements (42 ; 43) situés sur les deux parties (10 ; 20) sont des guidages en queue d'aronde et les extrémités sont conçues, au niveau de la pièce rapportée (41), de manière complémentaire à ces guidages en queue d'aronde.

8. Implant intervertébral (1) selon la revendication 7, **caractérisé en ce** les guidages en queue d'aronde se rétrécissent en partant des faces latérales ventrales (11 ; 21) jusqu'aux faces latérales dorsales (12 ; 22).

9. Implant intervertébral (1) selon l'une des revendications 1 à 8, **caractérisé en ce que** les moyens (40) comprennent deux parties de pièce rapportée (63), parallèles aux faces latérales sur le côté (13 ; 14 ; 23 ; 24), qui peuvent être mises en appui contre les surfaces opposées (16 ; 26).

10. Implant intervertébral (1) selon l'une des revendications 1 à 9, **caractérisé en ce que** la pièce rapportée (41) peut être fixée de manière amovible à l'une des deux parties (10 ; 20) au moyen d'une vis (44).

11. Implant intervertébral (1) selon l'une des revendications 1 à 10, **caractérisé en ce que** les parties supérieure et inférieure (10 ; 20) comprennent chacune au moins deux alésages (80) présentant des axes longitudinaux (83), traversant des faces latérales ventrales (11 ; 21) aux surfaces d'apposition (15 ; 25) pour recevoir des moyens de fixation osseuse (81).

12. Implant intervertébral (1) selon la revendication 11, **caractérisé en ce que** les axes longitudinaux (83) des alésages (80) forment un angle γ avec l'axe central (2).

13. Implant intervertébral (1) selon la revendication 12, **caractérisé en ce que** l'angle γ se situe dans une plage de 20° à 65°.

14. Implant intervertébral (1) selon l'une des revendications 11 à 13, **caractérisé en ce que** les axes longitudinaux (83) des alésages (80), vu depuis les faces latérales ventrales (11 ; 21), divergent depuis les surfaces internes (16 ; 26) jusqu'aux surfaces d'apposition (15 ; 25).

15. Implant intervertébral (1) selon l'une des revendications 11 à 14, **caractérisé en ce que** les alésages (80) se rétrécissent de manière conique vers les surfaces d'apposition (15 ; 25).

16. Implant intervertébral (1) selon l'une des revendications 11 à 15, **caractérisé en ce que** les alésages (80) présentent un taraudage (82).
